(19) Europäisches Patentamt
European Patent Office
Office européen des brevets

(11) Publication number: 0 213 082 B1

# (12) EUROPEAN PATENT SPECIFICATION

(45) Date of publication of the patent specification: 18.10.89

(51) Int. Cl.⁴: C07F 9/10, C07F 9/65, A61K 31/675

(21) Application number: 86810379.7

(22) Date of filing: 25.08.86

(54) **Substituted hydroxy-heterocyclyl-alkoxy phosphinyloxy trialkylaminium hydroxide inner salt oxides, processes for their preparation and their use as pharmaceuticals.**

(30) Priority: 30.08.85 US 771288
28.10.85 US 792095

(43) Date of publication of application: 04.03.87 Bulletin 87/10

(45) Publication of the grant of the patent: 18.10.89 Bulletin 89/42

(84) Designated Contracting States: AT BE CH DE FR GB IT LI LU NL SE

(56) References cited:
EP-A- 0 050 460
EP-A- 0 178 261
EP-A- 0 188 384
FR-A- 1 551 060

The file contains technical information submitted after the application was filed and not included in this specification

(73) Proprietor: SANDOZ AG, Lichtstrasse 35, CH-4002 Basel(CH)
(84) Designated Contracting States: BE CH FR GB IT LI LU NL SE

(73) Proprietor: SANDOZ-PATENT-GMBH, Humboldtstrasse 3, D-7850 Lörrach(DE)
(84) Designated Contracting States: DE

(73) Proprietor: SANDOZ-ERFINDUNGEN Verwaltungsgesellschaft m.b.H., Brunner Strasse 59, A-1235 Wien(AT)
(84) Designated Contracting States: AT

(72) Inventor: Houlihan, William J., 15 Raynold Road, Mountain Lakes, N.J. 07046(US)
Inventor: Lee, Mark Leonard, 84 Espanong Road, Lake Hopatcong, N.J. 07849(US)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to certain substituted hydroxy-heterocyclylalkoxyphosphinyloxy-trialkylaminium hydroxide inner salt oxides processes for their preparation, pharmaceutical compositions containing them and their use as pharmaceuticals in particular as anti-tumor agents.

The following disclosure is related to the present invention:

Takeda EP-A 50 460.

It discloses various tridecyloxy- or tetradecyloxy-propane derivatives with antitumor, antifungal and antiprotozoal activity.

Sankyo EP-A 188 384 discloses various phosphate ester derivatives as anticancer agents, in particular, in Tables 1 and 2, tetrahydrofuran derivatives structurally similar to the compounds of the present invention wherein $R_4$ and $R_5$ together with the adjacent carbon atom would represent a 3-tetrahydrofuran ring; such compounds have been deleted from the scope of the present invention. The compounds in EP 188 384 have a different structure to the remaining compounds of the present invention and EP 188 384 was not published before the priority date of the present invention.

More particularly the invention concerns compounds of formula I

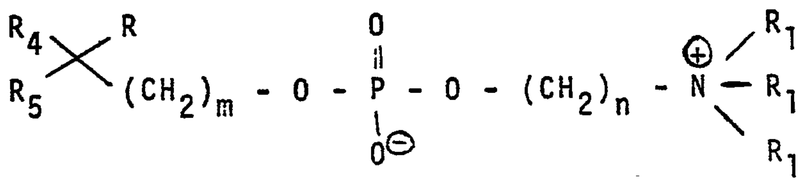

wherein

R represents hydrogen, $C_{14-18}$-alkyl or $CH_2OR_2$ or $CH_2CH_2OR_2$, wherein $R_2$ represents $C_{14-18}$-alkyl

each $R_1$ represents independently $C_{1-3}$ alkyl

m is 1 or 2

n is an integer from 2 to 8

and $R_4$ and $R_5$ together with the adjacent carbon atom represent

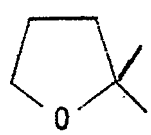

(a)

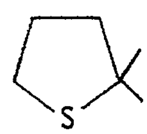

(b)

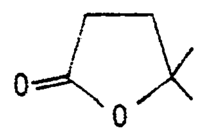

(c)

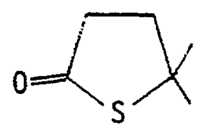

(d)

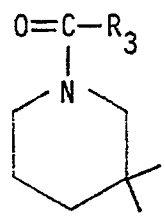

or (e)

wherein $R_3$ represents alkyl or alkoxy, each containing 12 to 24 carbon atoms

whereby when $R_4$ and $R_5$ together with the adjacent carbon represent

i) ring e) R is H and ii) other than ring e) R is other than H.

Examples of particular compound groups encompassed within the invention are:

Subclass Ia) wherein $R_4$ and $R_5$ together with the adjacent carbon atom represent ring a) or b), especially ring a);

Subclass Ib) wherein $R_4$ and $R_5$ together with the adjacent carbon atom represent ring c) or d), especially ring c);

Within these subclasses compounds are preferred, wherein R is R′ and represents n-$C_{16-18}$ alkyl, $CH_2OR_2'$ or $CH_2CH_2OR_2'$ wherein $R^{2'}$ is n-$C_{16-18}$ alkyl and n is an integer from 2 to 6 (sub-classes Ia′ etc). Especially preferred are compounds wherein R is R″ and represents n-$C_{16-18}$ alkyl or $CH_2OR_2'$ and n is an integer from 2 to 4 (subclasses Ia″ etc). Particularly preferred are compounds wherein R is R″ as defined above, m is 1, n is 2 and each $R_1$ is $CH_3$ (subclasses Ia‴ etc).

A further subclass of compounds is subclass Ie wherein $R_4$ and $R_5$ together with the adjacent carbon atom represent ring e). Within this subclass compounds are preferred wherein $R_3$ is $R_3'$ and represents alkyl or alkoxy, each with 12 to 20 carbon atoms m is 1 and n is an integer from 2 to 6 (subclass Ie'). Especially, preferred are compounds wherein $R_3$ is $R_3''$ and represents alkyl or alkoxy, each with 12 to 18 carbon atoms, m is 1 and n is an integer from 2 to 4 (sub-lass Ie''). Particularly preferred are compounds wherein $R_3$ is $R_3'''$ and represents alkoxy with 12 to 24, especially 12 to 18 carbon atoms and is preferably straight chained, m is 1 and n is an integer from 2 to 4 (subclass Ie'''). In the compounds of formula I and in each subclass it is preferred that each $R_1$ is the same.

Particularly preferred individual compounds are 2-[hydroxy(tetrahydro)-2-(octadecyl-5-oxo-2-furanyl)methoxyphosphinyloxy]N,N,N-trimethyl-ethanaminium hydroxide, inner salt-4-oxide
2-[(2-octadecyloxymethyltetrahydro-2-furanylmethoxy)-hydroxyphosphinyloxy]-N,N,N-trimethylethanaminium hydroxide, inner salt-4-oxide
2-hydroxy-[1-octadecyloxycarbonyl-3-piperidinyl-methoxy]-phosphinyloxy]-N,N,N-trimethylethanaminium hydroxide, inner salt-4-oxide.

The compounds according to the invention may be prepared by reacting a compound of formula C

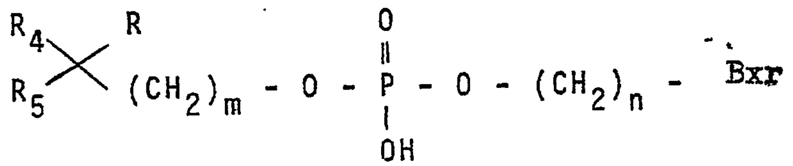

C

with a compound of formula CC

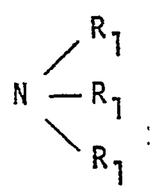

CC

wherein R, $R_1$, $R_4$, $R_5$, m and n are as defined above and X is a leaving group.

The reaction is suitably carried out in the presence of an inert organic solvent such as a lower alkanol e.g. methanol or ethanol, an aromatic hydrocarbon e.g. toluene or benzene, a dialkylamide such as dimethyl formamide; or acetonitrile at a temperature of e.g. 50° to 70° C. These conditions are particularly suited when employing a preferred leaving group namely bromine, iodine, tosyl or mesyl. The starting material of formula C wherein X is bromine may be prepared by reacting a compound of formula CCC

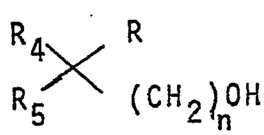

CCC

with a compound of formula CCCC

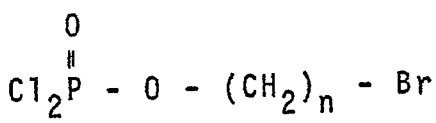

CCCC

The reaction is carried out in conventional manner e.g. in the presence of an amine base such as pyridine or triethylamine and in an inert organic solvent, e.g. an aromatic hydrocarbon such as toluene, benzene or xylene, a halogenated aliphatic hydrocarbon, such as methylene chloride, chloroform or carbon tetrachloride, a halogenated aromatic hydrocarbon, e.g. chlorobenzene, or an ether such as diethyl ether. The reaction may be carried out at temperatures of from 20° to 70°C.

The product thus produced is then subjected to basic hydrolysis, e.g., by suspending the product in water. The hydrolysis is conveniently carried out at a temperature of from 20° to 100°C.

The compounds of formula I wherein n is 2 may alternatively be prepared by reacting a compound of formula D

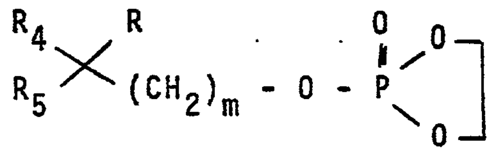

D

with a compound of formula CC

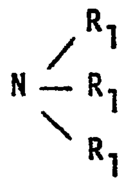

CC

wherein R, $R_1$, $R_4$, $R_5$ and m are as defined above.

The reaction is carried out essentially as described for the reaction of C with CC.

The starting materials of formula D, may be prepared by reacting a compound of formula CCC

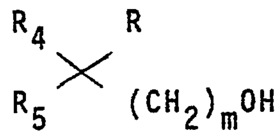

CCC

with a compound of formula DD

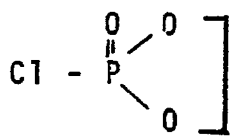

DD

This reaction is carried out in an inert organic solvent such as a halogenated hydrocarbon e.g. methylene chloride, chloroform or an aromatic hydrocarbon e.g. benzene, toluene and in the presence of a tertiary amine such as $C_{1-4}$ trialkylamine, e.g. triethylamine and optionally of a catalyst e.g. 4-dimethylaminopyridine at temperatures of e.g. 20° to 40°C.

The starting material of formula CCC may be prepared according to or analogously to the reaction shown in the following schemes.

LEGEND FOR REACTION SCHEMES

Bn = benzyl
M = alkali or alkaline earth metal
Y = O or S
Z = $CH_2$ or C=O
E = Cl, Br, I or toluenesulfonyl

Examples of conditions for the processes in the reaction schemes are given by way of illustration in the following tables or in the examples hereinafter.

ABBREVIATIONS

IO = inert organic
HC = hydrocarbon
HalHC = halogenated hydrocarbon
THF = tetrahydrofuran
DMSO = dimethylsulfoxide
DMA = dimethylacetamide
DMS = dimethylsulfide
DMF = dimethylformamide

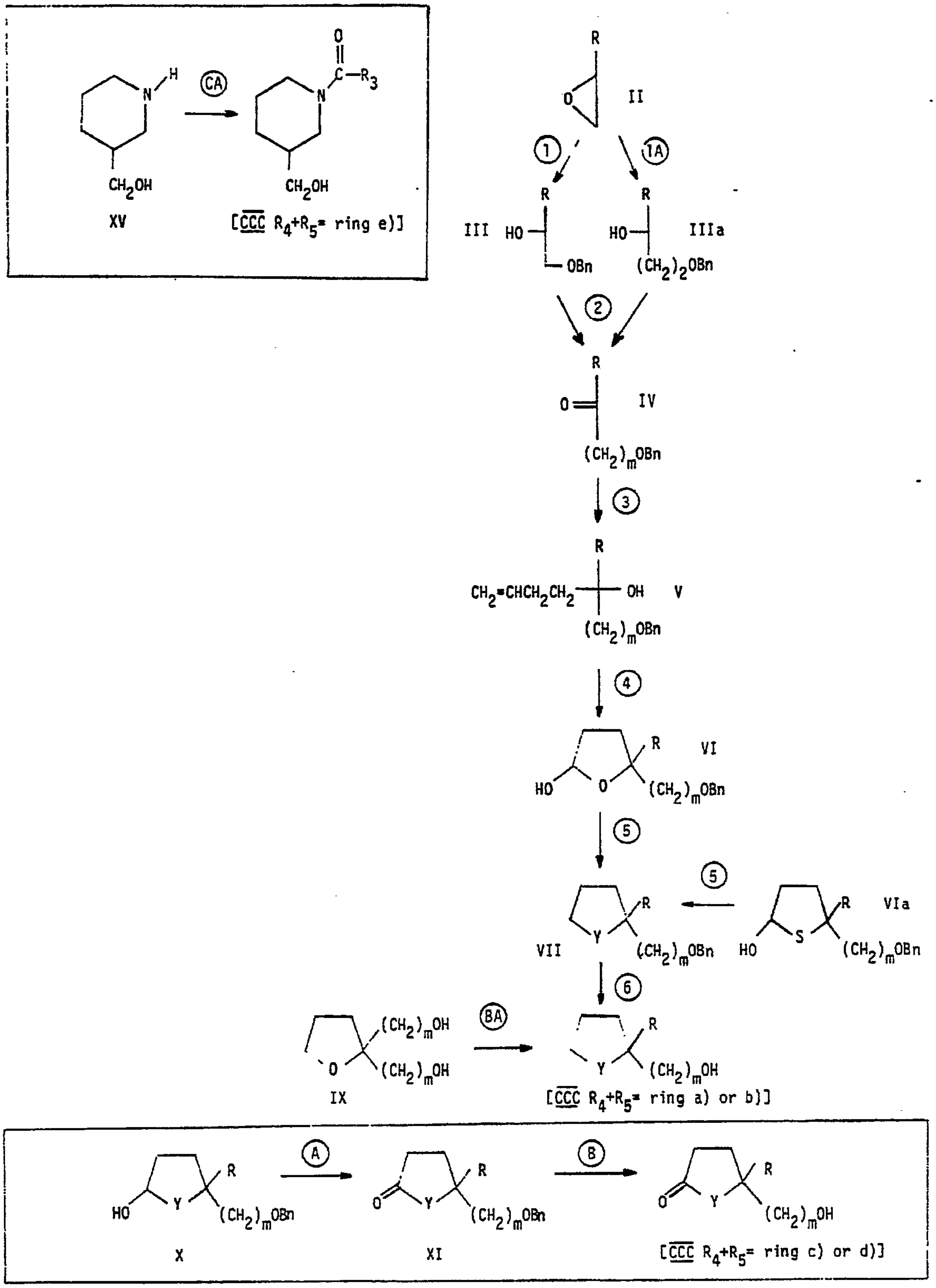


XV

[$\overline{CCC}$ $R_4$+$R_5$= ring e)]

II

III

IIIa

IV

V

VI

VIa

VII

IX

[$\overline{CCC}$ $R_4$+$R_5$= ring a) or b)]

X

XI

[$\overline{CCC}$ $R_4$+$R_5$= ring c) or d)]

| Reaction | Type/Steps | Special conditions/Reactants | Temperature | Solvent |
|---|---|---|---|---|
| 1 | | Add benzyl alcohol; MH preferably alkalimetal hydride esp. NaH | 30° to 100° | IO such as: dialkyl amide, e.g. DMF, DMA, aromatic HC e/g. toluene, benzene, xylene; cyclic ether e.g. THF |
| 1A | | Add benzyloxy methyllithium | −100° to −60° | IO such as: lower dialkylether e.g. diethylether or cyclic ether e.g. THF |
| 2 | oxidation | i) chromium based oxidant e.g. pyridinium chlorochromate | 20 to 40° | IO such as: HalCH e.g. $CH_2Cl_2$ |
| | | or | | |
| | | ii) a) anhydride e.g. $(CF_3CO)_2O$ or acid chloride, e.g. $(COCl)_2$ plus e.g. DMSO | −78 to 25° esp. −78° to 0° | IO such as: HalHC e.g. $CH_2Cl_2$ |
| | | b) $C_{1-3}$trialkylamine e.g. $(C_2H_5)_3N$ | 0 to 25° | aromatic HC e.g. toluene or xylene |
| 3 | | a) preparation of $CH_2{=}CHCH_2CH_2M$ (XX) from M e.g. Li or Mg + haloalkene e.g. 4-bromo-1-butene | 20 to 65° | aliphatic or cyclic ether e.g. THF |
| | | b) react XX with IV | −78 to 20° | aliphatic or cyclic ether e.g. THF or mixture e.g. with pentane or hexane |
| 4 | | a) $O_3$ in $O_2$ stream | −78 to −50° | as 2 (i) |
| | | b) add reducing agent e.g. trialkyl phosphite or dialkyl sulphide e.g. DMS | 20 to 30° | |
| 5 | | trialkyl silane e.g. $(C_2H_5)_3SiH$ + Lewis acid e.g. borontrifluoride etherate as catalyst | −30 to 0° | as 2 (i) |
| 6 | | Pd, PdOH, Pt/C as catalyst + $H_2$ at pressure of $1 \cdot 10^5$ to $4.5 \cdot 10^5$ Pa | 20 to 50° | lower alkanol e.g. $C_2H_5OH$, $CH_3OH$ + optionally 15% $H_2O$ |
| BA | | Add $R_2E$ XXI in the presence of MOH | 15° to 50° | IO such as dialkylamide e.g. DMF, DMA, aromatic HC; e.g. toluene, benzene, xylene or mixtures; or DMSO, cyclic ether e.g. THF or a mixture. |
| A | oxidation | When Y = O; as 2 | as 2 | as 2 |
| | | When Y = S; air or $O_2$ + Pt/C | 20° to 70° | cyclic ether e.g. dioxane |
| B | hydrogenolysis | as 6 | as 6 | as 6 |
| CA | | $E\overset{\overset{O}{\parallel}}{C}R_3$ in amine base e.g. pyridine or trialkylamine esp. triethylamine | −20 to 30° | IO such as aromatic HC e.g. benzene or toluene or aliphatic Hal HC e.g. $CHCl_3$, $CH_2Cl_2$ |

Intermediates containing substituents other than those described in the reaction schemes may be prepared analogously thereto or are either known or may be prepared analogously to known methods.

Starting materials set forth above are either known and/or may be obtained analogously to known methods in conventional manner.

Final products and intermediates may be isolated and purified in conventional manner. Intermediates,

where appropriate may be employed directly in the following step without purification.

As is evident to those skilled in the art, the compounds of formula I may exist in racemic or enantiomeric form and the invention is intended to cover all forms.

Enantiomeric forms may be recovered in conventional manner, e.g. by resolution of end or intermediate products or by employing optically active starting materials.

As indicated above, the compounds of formula I are indicated for use as anti-tumor agents and are, therefore, indicated for use in inhibiting the growth of various lymphomas, sarcomas, myelomas and leukemias. The anti-tumor activity of the compounds of formula I may be demonstrated employing the Tumor Cell Cytotoxicity test (TCC test) as follows:

In flat bottom microtiter plates (Nunc Roskilde, Denmark) were placed Abelson 8.1 lymphoma, YAC L1210, P815, Meth A fibrosarcoma or fresh human neuroblastoma tumor cells in DMEM + 10% fetal calf serum and the tumor cell-containing plates were incubated with 1.3 and 5 µg of the test compound for a period of 6 to 72 hours. The number of tumor cells present in the Abelson 8.1, YAC, L1210 and P815 assays was determined by measuring the alkaline phosphatase as follows:

The tumor cell plates were centrifuged (500 x g) for ten minutes and the supernatant flicked off. Without further washing, 100 µl of buffer containing 20 µl of diethanolamine, 2 µM of $MgCl_2 \cdot 6H_2O$, 2.5 µM of p-nitro-phenylphosphate and 10 mg Triton X–100 were added. The samples were incubated for 60 minutes at room temperature and the enzymatic reaction was terminated by the addition of 100 µl of 0.5 N NaOH. The absorbance was then measured at 405 nm using a Titertek Multiskan apparatus.

The number of tumor cells present in the Meth A fibrosarcoma and human neuroblastoma assays was measured by $^3H$-thymidine uptake as follows:

After 72 hours, the cells are thoroughly washed, and each well treated with ca. 0.1 µC $^3H$-thymidine. After 4–6 hours, the cells are collected using a commercial cell harvester, and the radioactivity in the filtrate is measured in a scintillation counter.

The anti-tumor activity of the compounds of formula I may also be demonstrated employing the Influence on Cytotoxicity of ET–18–$OCH_3$ test as follows:

Bone marrow cell macrophages ($10^5$/well) obtained from (BALB/CX57/$BL_6$)FI mice were incubated with 10 µg of 1-octadecyl-2-methoxy-3-phosphoryl choline (ET–18–$OCH_3$) for 24 hours in flat bottom microtiter plates (Nunc Roskilde, Denmark), after which time they are centrifuged and washed once. Abelson 8.1 tumor cells in DMEM + 10% fetal calf serum and 1, 3 and 5 µg of the test compound were then added to the plates. With the cytotoxicity of ET–18–$OCH_3$ (10 µg) alone set at 100%, the inhibition or enhancement of the cytotoxic effect, as measured by an alkaline phosphatase assay, was determined and values recorded after 72 hours for 1, 3 and 5 µg of the test substance.

The usefulness of the compounds of formula I in treating tumors may additionally be demonstrated employing the following procedure:

Meth A fibrosarcoma cells were induced in BALB/C mice by administering methylcholanthrene according to the procedure of Old, et al. (L.J.Old, E.A.Boyse, D.A.Clarke and E.Carswell, Ann.N.Y.Acad.Sci., 101, 80 (1962).

These tumor cells were harvested from the peritoneal cavity 10 to 12 days after administration of methylcholanthrene. Ten $CBF_I$ mice of 10–12 week age were each implanted with 7.3 x $10^6$ Meth A sarcoma cells to serve as control. A second group of ten $CBF_1$ mice were each implanted with 7.3 x $10^6$ Meth A sarcoma cells and on day one after implant each mouse was treated p.o. with 5–50 µg of the test compound per day for a total of twenty or twenty-seven days. Tumor growth and survivors were assayed on days 7, 14, 21 and 28 after tumor implantation.

The compounds are thus indicated for use as anti-tumor agents and an indicated suitable daily dosage for this use is from about 500 to 2000 mg preferably 1000 to 1500 mg suitably administered in divided dosages of 125 to 2000 mg preferably 250 to 1500 mg one to four times daily or in controlled release form or e.g. 20 mg/kg i.v. over a 24 hr period. A typical oral dosage form contains 300 to 600 mg (unit dose especially 300 to 500 mg in particular 350 to 450 mg).

The invention therefore also concerns compounds of formula I for use as pharmaceuticals e.g. in treating tumors.

The compounds may be administered alone, or in admixture with a pharmaceutically acceptable diluent or carrier, and, optionally other excipients, and administered orally in such forms as tablets, dispersible powders, granules, elixirs, capsules or suspensions or parenterally in such forms as sterile injectable solutions or suspensions.

The preferred pharmaceutical compositions from the standpoint of ease of preparation and administration are solid compositions, particularly tablets and hard-filled or liquid-filled capsules.

Such compositions also form part of the invention.

The following Examples, in which all temperatures are in °C illustrate the invention.

EXAMPLE 1:

2-[hydroxy(tetrahydro)-2-(octadecyloxymethyl-5-oxo-2-furanyl)methoxyphosphinyloxy]N,N,N-trimethyl-ethanaminium hydroxide, inner salt-4-oxide
(Subclass Ib; ring c), R = $CH_2OC_{18}H_{37}$; each $R_1 = CH_3$; n = 2, m = 1)

a) Preparation of 1–0-octadecyl-3-0-benzylglycerol

To 12.0 g of 60% sodium hydride in mineral oil (30 mmol; washed free of oil by the use of petroleum ether) was added 32.4 g (300 mmol) of benzyl alcohol in 200 ml of dry dimethylformamide. The suspension was heated under a flow of nitrogen to 80°C and maintained at this temperature for 45 minutes, after which time 63.2 g (0.19 mol) of an epoxide (prepared by the peracid oxidation of allyloctadecylether) in 100 ml of dimethyl formamide was added and the temperature maintained at 80°C for 15 hours. The solvent was then removed in vacuo and the residue chromatographed on silica gel employing a mixture of petroleum ether and diethyl ether in a ratio of 3:2 as the eluent to yield a low-melting waxy solid.

b) Preparation of 1-O-octadecyl-3-O-benzyldihydroxyacetone

To a complex prepared at –60°C from the addition of 10.4 ml of dry dimethyl sulfoxide to 8.9 g (70 mmol) of oxalyl chloride in 150 ml of methylene chloride, was added, dropwise, 27.0 g (62.2 mmol) of the compound prepared in a) above. After stirring the mixture under a nitrogen atmophere for 1 hour, 50 ml of triethylamine in 50 ml of methylene chloride was added and the resultant mixture was allowed to warm to 0°C over a period of 30 minutes, after which time it was quenched with 75 ml of water. After the mixture was allowed to warm to room temperature, the organic layer was separated, washed with a saturated sodium chloride solution, dried over magnesium sulfate and concentrated in vacuo to afford an oil which solidified on standing. Flash chromatography on silica gel employing a mixture of petroleum ether and diethyl ether in a ratio of 7:3 as the eluent yielded a white solid.

c) Preparation of 2-benzyloxymethyl-2-hydroxy-1-octadecyloxyhex-5-ene

1.12 g (8 mmol) of 4-bromo-1-butene in 15 ml of dry ether was reacted with 200 mg of magnesium turnings at reflux under a nitrogen atmosphere for 1 hour. The resulting Grignard reagent was cooled to –60°C and then treated with 2.16 g (5 mmol) of the compound prepared in b) above in 30 ml of ether. After 1 hour at –60°C, the mixture was warmed overnight to room temperature and then quenched with a saturated aqueous ammonium acetate solution and partitioned. The organic layer was then washed twice with ammonium acetate, washed with a saturated sodium chloride solution and dried over magnesium sulfate, after which time the solvent was removed under reduced pressure. Purification of the crude product was effected on silica gel employing a mixture of petroleum ether and diethyl ether as the eluent to yield a colorless oil.

d) Preparation of 5-benzyloxymethyl-2-hydroxy-5-octadecyloxymethyltetrahydrofuran

6.0 g (12.3 mmol) of the compound prepared in c) above in 100 ml of methylene chloride was treated with ozone at –60°C. After consumption of the olefin, 25 ml of dimethylsulfide was added and the mixture allowed to warm to room temperature. The solvent was removed in vacuo and the crude product was purified on silica gel employing a mixture of petroleum ether and diethylether in a ratio of 7:3 as the eluent to yield a colorless oil.

e) Preparation of 5-benzyloxymethyl-5-octadecyl-oxymethyl-4,5-dihydro-2(3H)furanone

To a solution of 1.6 g (7.4 mmol) of pyridinium chlorochromate in 25 ml of methylene chloride under a nitrogen atmosphere, was added 13.2 g (6.5 mmol) of the compound prepared in d) above in 25 ml of methylene chloride. After 18 hours at 25°C, the solution was diluted with 150 ml of ether, filtered through silica gel and the filtrate evaporated to afford an oil. The oil was then flash chromatographed on silica gel employing a mixture of petroleum ether and diethyl ether in a ratio of 7:3 as the eluent to yield a colorless oil.

f) Preparation of 5-hydroxymethyl-5-octadecyloxymethyl-4,5-dihydro-2(3H) furanone

A mixture containig 10.7 g (21.83 mmol) of the compound prepared in e) above, 300 ml of a mixture of ethyl alcohol and water in a ratio of 9:1 and 1.5 g of 5% palladium on carbon (50% water content) was placed in a pressure bottle and hydrogenated at 40°C under a pressure of $3.45 \cdot 10^5$ Pa complete. The catalyst was then filtered off and the filtrate concentrated in vacuo. The residue was crystallized from methanol to yield a solid.

g) Preparation of the title compound

To 860 mg (2.16 mmol) of the compound prepared in f) above, 29 mg (0.24 mmol) of 4-dimethylaminopyridine and 0.42 ml (3 mmol) of triethylamine in 10 ml of benzene, was added 455 mg (3.2 mmol) of 2-chloro-2-oxo-1,3,2-dioxaphospholane. The resultant mixture was allowed to stir at room temperature for 18 hours, after which time it was filtered and the filtrate was evaporated to dryness. The residue was taken up with 10 ml of dry acetonitrile, cooled to allow introduction of excess condensed trimethylamine, and then heated in a sealed tube under pressure for 20 hours at 70°C. The reaction mixture was then cooled to room temperature, diluted with an equal volume of acetonitrile and filtered to afford a white solid which was recrystallized from a mixture of methylene chloride and acetone to yield a solid, m.p. 215°–220°C (dec.).

EXAMPLE 2:

2-(hydroxy(tetrahydro)-2-(octadecyl-5-oxo-2-furanyl)methoxyphosphinyloxy]-N,N,N-trimethylethanaminium hydroxide, inner salt -4-oxide
(Subclass Ib; ring c),R = $C_{18}H_{37}$; m = 1, n = 2, each $R_1 = CH_3$) analogous to example 1 m.p. 235°–240°C.

EXAMPLE 3:

2-[hydroxy-[tetrahydro-2-(2-octadecyl-oxyethyl)-5-oxo-2-furanyl]-methoxy-phosphinyloxy)-N,N,N-trimethyl ethanaminium hydroxide, inner salt -4-oxide
(Subclass Ib; ring C), R = $CH_2CH_2OC_{18}H_{37}$; m = 1, n = 2, each $R_1 = CH_3$) analogous to example 1 m.p. 107° (dec.).

EXAMPLE 4:

2-[hydroxy-2-[tetrahydro-2-(octadecyl-oxymethyl)-5-oxo-2-furanyl]-ethoxy-phosphinyloxy]-N,N,N-trimethyl ethanaminium hydroxide, inner salt -4-oxide
(Subclass Ib; ring c), R = $CH_2OC_{18}H_{37}$; m = 2, n = 2, each $R_1 = CH_3$) analogous to example 1 m.p. 145°C (dec.).

EXAMPLE 5:

2-[(2-octadecyloxymethyltetrahydro-2-furanylmethoxy)-hydroxyphosphinyl-oxy]-N,N,N-trimethylethanaminium hydroxide inner salt -4-oxide
(Subclass Ia; ring a), R = $CH_2OC_{18}H_{37}$, m = 1, n = 2, each $R_1 = CH_3$).

a) Preparation of tetrahydro-2-octadecyloxymethyl-2-furanmethanol

To a mixture of 2.64 g (0.020 mol) of 2,2-bis-(hydroxymethyl)-tetrahydrofuran and 2.2 g (0.0066 mol) of bromooctadecane in 8 ml of 1:1 mixture of dimethylsulfoxide and tetrahydrofuran was added 1.84 g (0.0264 mol) of finely powdered potassium hydroxide. The resultant mixture was then stirred at room temperature for 2 hours, after which time it was poured onto 100 ml of water, diluted with 20 ml of an aqueous saturated sodium chloride solution and extracted with ether. The ether extract was then washed with an aqueous saturated sodium chloride solution, dried over anhydrous sodium sulfate and the ether was removed under reduced pressure. The crude product was then purified by column chromatography on silica gel employing a mixture of methyl-t-butyl ether and hexane (3:1 ratio) as the eluent to yield the desired compound as a wax.

The title compound is prepared analogously to the last step of example 1, white solid m.p. 232–235°C.

EXAMPLE 6:

2-hydroxy-[1-octadecyloxycarbonyl-3-piperidinylmethoxy]-phosphinyloxy]-N,N,N-trimethylethanaminium hydroxide inner salt -4-oxide
(Subclass Ie; $R_3 = OC_{18}H_{37}$; m = 1; n = 2; each $R_1 = CH_3$)

a) Preparation of 3-hydroxymethyl-N-octadecyloxycarbonyl piperidine

To a solution of 10.52 g (91 mmol) of 3-piperidinemethanol in 100 ml of dichloromethane, and 13.3 ml (96 mmol) of triethylamine was added, at room temperature, a solution of 87 mmol of octadecylchloroformate in toluene. The resultant mixture was then allowed to react for 24 hours, after which time it was washed with water. The aqueous layer was then extracted twice with dichloromethane and the organic layers were combined, dried with magnesium sulfate and filtered. The solvent was then removed in vacuo to yield the crude product as a solid. The crude product was then chromatographed on silica gel employing dichloromethane as the eluent to yield a white solid.

Preparation of the title compound

0.5 g (1.22 mmol) of the compound prepared in a) above was dissolved in 20 ml of benzene containing 15 mg of N,N-dimethylaminopyridine and 0.21 ml (1.52 mmol) of triethylamine. To the mixture was added 0.23 g (1.62 mmol) of 2-chloro-2-oxo-1,3,2-dioxaphospholane and the resultant mixture was stirred at room temperature for 2 hours. The salts were then removed by filtration and the residue taken up in dry acetonitrile which was then cooled to −78°C. Trimethylamine was then condensed therein, the reaction vessel was capped, warmed to 60°C and maintained at this temperature for 24 hours. The reaction mixture was then cooled in an ice-methanol bath and the solids were then isolated by filtration, washed with acetonitrile and dried under vacuum to yield the crude product. The crude product was then chromatographed on silica gel employing a mixture of chloroform, methanol and water (in a 2:1:0.2 ratio) as the eluent to yield a white solid, m.p., >230°C

EXAMPLE 7:

2-hydroxy-[1-hexadecyloxycarbonyl-3-piperidinylmethoxy]-phosphinyloxy]-N,N,N-trimethylethaneaminium hydroxide inner salt -4-oxide
(Subclass Ie; $R_3 = OC_{16}H_{33}$, m = 1, n = 2, each $R_1 = CH_3$) analogous to example 9, white solid m.p. 230°C.

EXAMPLE 8:

2-hydroxy-[1-hexadecanoyl-3-piperidinylmethoxy]-phosphinyloxy]-N,N,N-trimethylethanaminiumhydroxide inner salt -4-oxide
(Subclass Ie; $R_3 = C_{15}H_{31}$, m = 1, n = 2, each $R_1 = CH_3$) analogous to example 9, white solid; m.p. 229°.

EXAMPLE 9:

6-hydroxy-[1-octadecyloxycarbonyl-3-piperidinylmethoxy]-phosphinyloxy]-N,N,N-trimethylhexanaminium hydroxide inner salt -8-oxide
(Subclass Ie; $R_3 = OC_{18}H_{37}$, m = 1, n = 6, each $R_1 = CH_3$)

To 10 ml of dry benzene containing 0.358 g (1.2 mmol) of 6-bromohexyloxyphosphorodichloridate was added 0.411 g (1.0 mmol) of the compound of Example 1a) and the resultant mixture was cooled in an ice-salt bath, after which time it was treated dropwise, under a nitrogen atmosphere, with a solution of 103yl of pyridine in 1 ml of benzene.

The ice-salt bath was then removed and the mixture allowed to warm to room temperature over a period of 6 hours. The volatiles were then removed under reduced pressure and the residue was suspended in 15 ml of water and heated in the steam bath for 1 hour. The mixture was then cooled to room temperature, extracted with chloroform and the extracts dried over magnesium sulfate, filtered and the solvent removed <u>in vacuo</u>. The residue was then treated with trimethylamine in a manner analogous to that described above in the last step in the preparation of Example 1 to afford the hydrobromide salt of the title compound. The hydrobromide salt was then taken up in 10 ml of methanol into which 0.310 g of silver carbonate was suspended. After 90 minutes, the solids were removed by filtration and the filtrate concentrated <u>in vacuo</u> to yield the crude product. The crude product was then chromatographed on silica gel employing a mixture of chloroform, methanol and water (in a 2:1:0.2 ratio) as the eluent to yield a white solid, m.p. 137°–140°C.

**Claims for the Contracting States: BE, CH, DE, FR, GB, IT, LI, LU, NL, SE**

1. Compounds of formula I

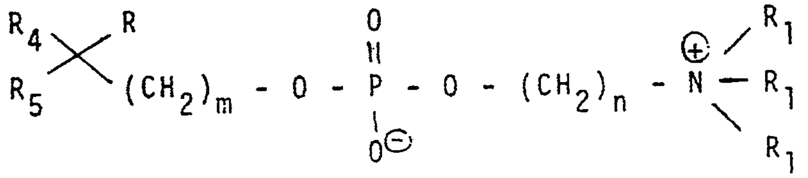

wherein
R represents hydrogen, $C_{14-18}$-alkyl or $CH_2OR_2$ or $CH_2CH_2OR_2$, wherein $R_2$ represents $C_{14-18}$alkyl
each $R_1$ represents independently $C_{1-3}$ alkyl
m is 1 or 2
n is an integer from 2 to 8
and $R_4$ and $R_5$ together with the adjacent carbon atom represent

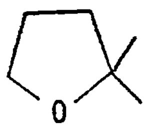

(a)

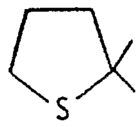

(b)

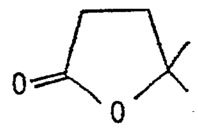

(c)

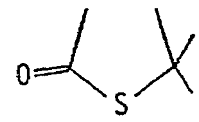

(d)

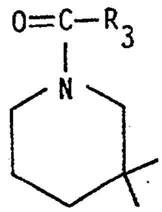

or (e)

wherein $R_3$ represents alkyl, alkenyl, alkynyl, alkoxy, alkenoxy or alkynoxy each containing l2 to 24 carbon atoms

whereby when $R_4$ and $R_5$ together with the adjacent carbon represent (i) ring e) R is H and (ii) other than ring e) R is other than H.

2. A compound according to claim I wherein each $R_1$ is the same and $R_4$ and $R_5$ together with the adjacent carbon atom represent ring a); b); c) or d).

3. A compound according to claim I wherein each $R_1$ is the same and $R_4$ and $R_5$ together with the adjacent carbon atom represent ring e) and m is l.

4. A compound according to claim I selected from those wherein $R_4$ and $R_5$ together with the adjacent carbon atom represent ring a); b); c) or d) and

i) R is R' and represents n-$C_{16-18}$alkyl, $CH_2OR_2'$ or $CH_2CH_2OR_2'$ wherein $R_2'$ is n-$C_{16-18}$ alkyl and n is an integer from 2 to 6

ii) R is R″ and represents n-$C_{16-18}$alkyl or $CH_2OR_2'$ wherein $R_2'$ is as defined under (i) and n is an integer from 2 to 4,

iii) R is as defined under (ii), m is 1, n is 2 and each $R_1$ is $CH_3$; or

$R_4$ and $R_5$ together with the adjacent carbon atom represent ring e) and

i) $R_3$ is $R_3'$ and represents alkyl or alkoxy, each with 12 to 20 carbon atoms, m is 1 and n is an integer from 2 to 6,

ii) $R_3$ is $R_3''$ and represents alkyl or alkoxy, each with 12 to 18 carbon atoms, m is 1 and n is an integer from 2 to 4,

iii) $R_3$ is $R_3'''$ and represents alkoxy with 12 to 18 carbon atoms, m is 1 and n is an integer from 2 to 4, and the other symbols are as defined in claim 1.

5. 2-[Hydroxy-(tetrahydro)-2-(octadecyl-5-oxo-2-furanyl)methoxy-phosphinyloxy]-N,N-N-trimethylethanaminium hydroxide, inner salt-4-oxide.

6. 2-[(2-Octadecyloxymethyltetrahydro-2-furanylmethoxy)-hydroxyphosphinyloxy]-N,N,N-trimethylethanaminium hydroxide inner salt-4-oxide.

7. 2-Hydroxy-[1-octadecyloxycarbonyl-3-piperidinyl-methoxy]-phosphinyloxy]-N,N,N-trimethylethanaminium hydroxide inner salt-4-oxide.

8. A pharmaceutical composition comprising a compound according to any one of claims 1 to 7 together with a pharmaceutically acceptable carrier or diluent.

9. A compound according to any one of claims 1 to 7 for use as a pharmaceutical.

10. A compound according to any one of claims 1 to 7 for use as an anti-tumor agent.

11. A process for preparing a compound according to claim 1 which comprises

a) reacting a compound of formula C

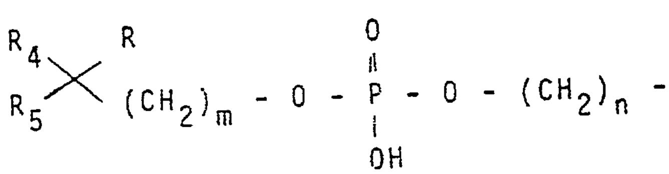

C

with a compound of formula CC

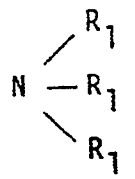

CC

wherein R, $R_1$, $R_4$, $R_5$, m and n are as defined above and X is a leaving group, or

b) when n is 2

reacting a compound of formula D

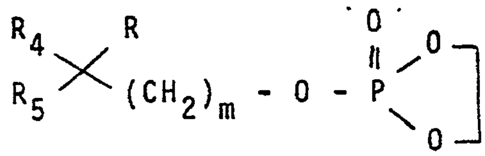

D

with a compound of formula CC

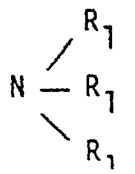

CC

wherein R, $R_1$, $R_4$, $R_5$ and m are as defined above.

**Claims for the Contracting State AT**

1. A process for the preparation of compounds of formula I

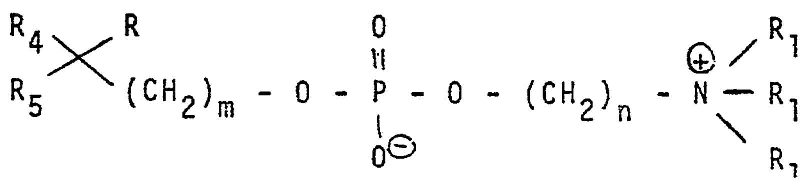

wherein

R represents hydrogen, $C_{14-18}$-alkyl or $CH_2OR_2$ or $CH_2CH_2OR_2$, wherein $R_2$ represents $C_{14-18}$alkyl

each $R_1$ represents independently $C_{1-3}$ alkyl

m is 1 or 2

n is an integer from 2 to 8

and $R_4$ and $R_5$ together with the adjacent carbon atom represent

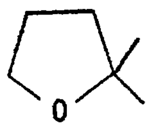
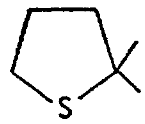
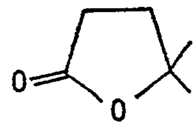
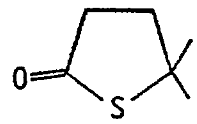

(a) (b) (c) (d)

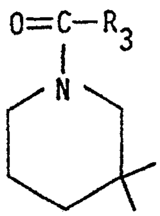

or (e)

wherein $R_3$ represents alkyl or alkoxy, each containing 12 to 24 carbon atoms whereby when $R_4$ and $R_5$ together with the adjacent carbon represent (i) ring e) R is H and (ii) other than ring e) R is other than H.

which comprises

a) reacting a compound of formula C

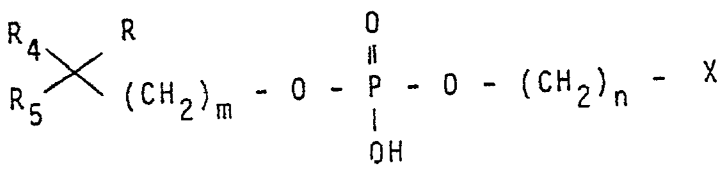

C

with a compound of formula CC

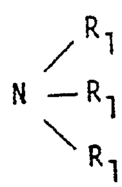

CC

wherein R, $R_1$, $R_4$, $R_5$, m and n are as defined above and X is a leaving group,

or

b) when n is 2

reacting a compound of formula D

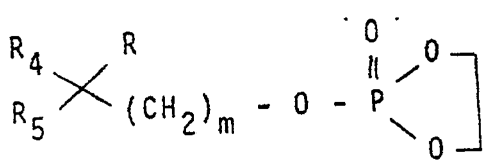

D

with a compound of formula CC

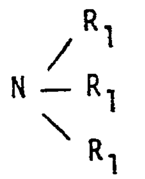

CC

wherein R, $R_1$, $R_4$, $R_5$ and m are as defined above.

2. A process according to claim 1 for the preparation of 2-[(2-octadecyloxymethyltetrahydro-2-furanylmethoxy)-hydroxyphosphinyloxy]-N,N,N-trimethylethanaminium hydroxyde inner salt-4-oxide which comprises

a) reacting a compound of formula C wherein
R is octadecyloxymethyl,
m is 1,
n is 2,
$R_4$ and $R_5$ together with the adjacent carbon atom represent a group a) and
X is a leaving group,
with a compound of formula CC wherein $R_1$ is methyl, or
b) reacting a compound of fromula D wherein R, m, $R_4$ and $R_5$ are as defined in this claim, with a compound of formula CC wherein $R_1$ is methyl.

3. A process for the production of the compound of claim 2 which comprises reacting the compound of formula D wherein R, m, $R_4$ and $R_5$ are as defined in claim 2, with trimethylamine.

4. A process for the production of a pharmaceutical composition which comprises mixing a compound of formula I as defined in claim 1 with a pharmaceutically acceptable carrier or diluent.

**Patentansprüche für die Vertragsstaaten BE, CH, DE, FR, GB, IT, LI, LU, NL, SE**

1. Verbindungen der Formel I

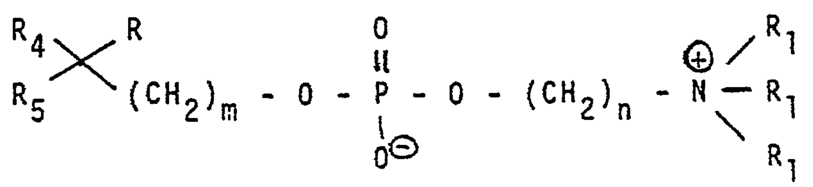

worin
R für Wasserstoff, $C_{14-18}$-Alkyl oder $CH_2OR_2$ oder $CH_2CH_2OR_2$, worin $R_2$ $C_{14-18}$Alkyl bedeutet, steht,
jedes $R_1$ unabhängig voneinander $C_{1-3}$Alkyl bedeutet,
m für 1 oder 2 steht,
n eine ganze Zahl von 2 bis 8 bedeutet und
$R_4$ und $R_5$ zusammen mit dem danebenliegenden Kohlenstoffatom stehen für:

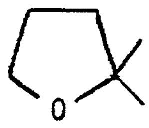 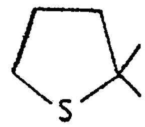 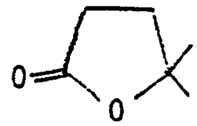 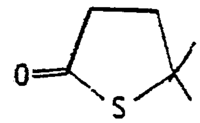

(a) (b) (c) (d)

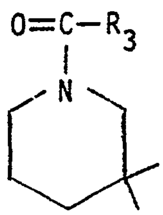

oder (e)

worin $R_3$ für Alkyl oder Alkoxy mit je 12 bis 24 Kohlenstoffatomen steht, wobei, falls $R_4$ und $R_5$ zusammen mit dem danebenliegenden Kohlenstoffatom stehen (i) für den Ring (e), R Wasserstoff bedeutet, und (ii) für einen anderen Ring als Ring (e), R nicht Wasserstoff bedeutet.

2. Eine Verbindung gemäß Anspruch 1, in der beide $R_1$ identisch sind und $R_4$ und $R_5$ zusammen mit dem danebenliegenden Kohlenstoffatom den Ring a); b); c) oder d) darstellen.

3. Eine Verbindung gemäß Anspruch 1, worin beide $R_1$ identisch sind und $R_4$ und $R_5$ zusammen mit dem danebenliegenden Kohlenstoffatom für den Ring e) stehen und m die Zahl 1 bedeutet.

4. Eine Verbindung gemäß Anspruch 1, ausgewählt aus den Verbindungen, in denen $R_4$ und $R_5$ zusammen mit dem danebenliegenden Kohlenstoffatom den Ring a); b); c) oder d) darstellen und
i) R ist R′ und stellt dar: n-$C_{16-18}$alkyl, $CH_2OR_2'$ oder $CH_2CH_2OR_2'$, worin $R_2'$ für n-$C_{16-18}$ Alkyl steht und n eine ganze Zahl von 2 bis 6 bedeutet,
ii) R ist R″ und stellt dar: n-$C_{16-18}$ Alkyl oder $CH_2OR_2'$, worin $R_2'$ die unter (i) angegebene Bedeutung besitzt und n für eine ganze Zahl von 2 bis 4 steht,
(iii) R die unter (ii) angegebene Bedeutung besitzt, m für 1, n für 2 und jedes $R_1$ $CH_3$ bedeutet; oder
$R_4$ und $R_5$ zusammen mit dem danebenliegenden Kohlenstoffatom den Ring e) darstellen und
i) $R_3$ ist $R_3'$ und stellt dar: Alkyl oder Alkoxy mit je 12 bis 20 Kohlenstoffatomen, m für 1 und n für eine ganze Zahl von 2 bis 6 stehen,
ii) $R_3$ ist $R_3''$ und stellt dar: Alkyl oder Alkoxy mit je 12 bis 18 Kohlenstoffatomen, m für 1 und n für eine ganze Zahl von 2 bis 4 stehen,
(iii) $R_3$ ist $R_3'''$ und stellt dar: Alkoxy mit 12 bis 18 Kohlenstoffatomen, m für 1 und n für eine ganze Zahl von 2 bis 4 stehen,
und die übrigen Symbole die in Anspruch 1 angegebene Bedeutung besitzen.

5. 2-[Hydroxy-(tetrahydro)-2-octadecyl-5-oxo-2-furanyl)-methoxy-phosphinyloxy]-N,N,N-trimethyl-ethanaminiumhydroxid-innersalz-4-oxid.

6. 2-[(2-Octadecyloxymethyltetrahydro-2-furanylmethoxy)-hydroxyphosphinyloxy]-N,N,N-trimethyl-ethanaminiumhydroxid-innersalz-4-oxid.

7. 2-Hydroxy-[1-octadecyloxycarbonyl-3-piperidinylmethoxy]-phosphinyloxy]-N,N,N-trimethylethanaminium hydroxid-innersalz-4-oxid.

8. Eine pharmazeutische Zusammensetzung, enthaltend eine Verbindung gemäß irgendeinem der Ansprüche 1 bis 7, zusammen mit einem pharmazeutisch verträglichen Träger- oder Verdünnungsmittel.

9. Eine Verbindung gemäß irgendeinem der Ansprüche 1 bis 7 zur Verwendung als Pharmazeutikum.

10. Eine Verbindung gemäß irgendeinem der Ansprüche 1 bis 7 zur Verwendung als antitumorales Mittel.

11. Ein Verfahren zur Herstellung einer Verbindung gemäß Anspruch 1, das beinhaltet
a) Umsetzung einer Verbindung der Formel C

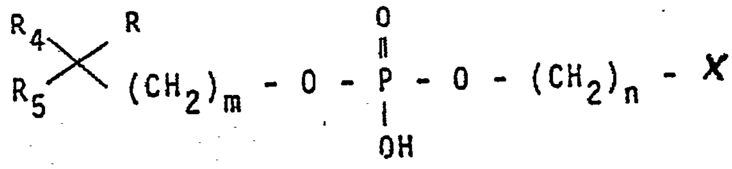

C

mit einer Verbindung der Formel CC

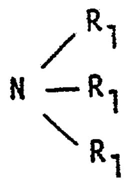

CC

worin R, $R_1$, $R_4$, $R_5$, m und n die oben angegebene Bedeutung besitzen und X für eine Abgangsgruppe steht,
oder
b) falls n 2 bedeutet,
Umsetzung einer Verbindung der Formel D

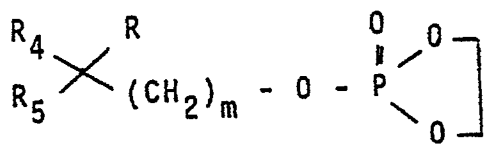

D

mit einer Verbindung der Formel CC

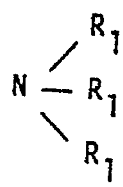

CC

worin R, $R_1$, $R_4$, $R_5$ und m die oben angegebene Bedeutung besitzen.

**Patentansprüche für den Vertragsstaat AT**

1. Ein Verfahren zur Herstellung von Verbindungen der Formel I

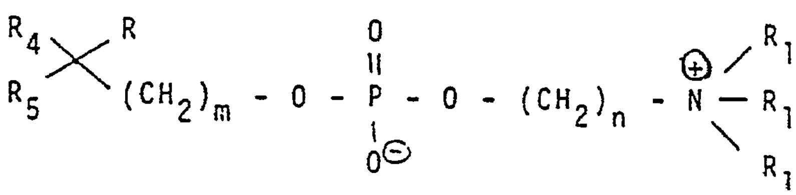

worin
R für Wasserstoff, $C_{14-18}$-Alkyl oder $CH_2OR_2$ oder $CH_2CH_2OR_2$, worin $R_2$ $C_{14-18}$Alkyl bedeutet, steht,
jedes $R_1$ unabhängig voneinander $C_{1-3}$Alkyl bedeutet,
m für 1 oder 2 steht,
n eine ganze Zahl von 2 bis 8 bedeutet und
$R_4$ und $R_5$ zusammen mit dem danebenliegenden Kohlenstoffatom stehen für:

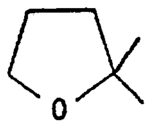 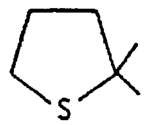 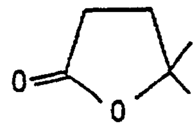 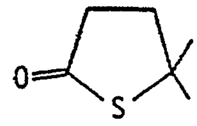

(a) (b) (c) (d)

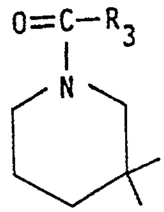

oder (e)

worin $R_3$ für Alkyl oder Alkoxy mit je 12 bis 24 Kohlenstoffatomen steht, wobei, falls $R_4$ und $R_5$ zusammen mit dem danebenliegenden Kohlenstoffatom stehen (i) für den Ring (e), R Wasserstoff bedeutet, und (ii) für einen anderen Ring als Ring (e), R nicht Wasserstoff bedeutet, das beinhaltet

a) Umsetzung einer Verbindung der Formel C

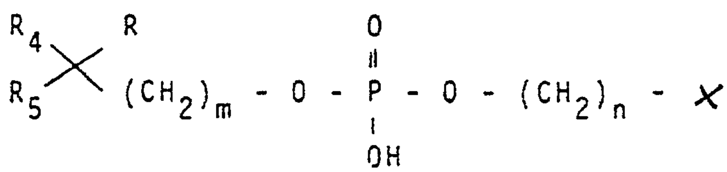

C

mit einer Verbindung der Formel CC

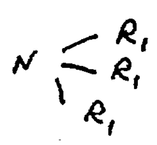

CC

worin R, $R_1$, $R_4$, $R_5$, m und n die oben angegebene Bedeutung besitzen und X für eine Abgangsgruppe steht,

oder

b) falls n 2 bedeutet,

Umsetzung einer Verbindung der Formel D

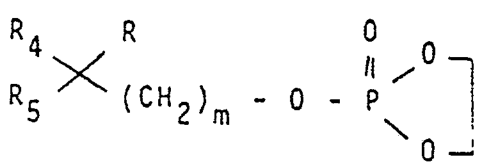

D

mit einer Verbindung der Formel CC

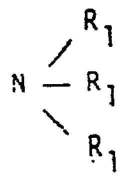

CC

worin R, $R_1$, $R_4$, $R_5$ und m die oben angegebene Bedeutung besitzen.

2. Ein Verfahren gemäß Anspruch 1 zur Herstellung von 2-[(2-Octadecyloxymethyltetrahydro-2-furanylmethoxy)-hydroxyphosphinyloxy]-N,N,N-trimethylethanaminiumhydroxid-innersalz-4-oxid, das beinhaltet

a) Umsetzung einer Verbindung der Formel C, worin
R Octadecyloxymethyl bedeutet,
m für 1 steht,
n 2 bedeutet,
$R_4$ und $R_5$ zusammen mit dem danebenliegenden Kohlenstoffatom eine Gruppe a) darstellen und X für eine Abgangsgruppe steht,
mit einer Verbindung der Formel CC, worin $R_1$ Methyl bedeutet,
oder
b) Umsetzung einer Verbindung der Formel D, worin R, m, $R_4$ und $R_5$ die in diesem Anspruch angegebene Bedeutung besitzen, mit einer Verbindung der Formel CC, worin $R_1$ Methyl bedeutet.

3. Ein Verfahren zur Herstellung der Verbindung des Anspruches 2, das die Umsetzung der Verbindung der Formel D, worin R, m, $R_4$ und $R_5$ die in Anspruch 2 angegebene Bedeutung besitzen, mit Trimethylamin beinhaltet.

4. Ein Verfahren zur Herstellung einer pharmazeutischen Zusammensetzung, das das Mischen einer Verbindung der Formel I, wie in Anspruch 1 definiert, mit einem pharmazeutisch verträglichen Träger- oder Verdünnungsmittel beinhaltet.

**Revendications pour les Etats Contractants BE, CH, DE, FR, GB, IT, LI, LU, NL, SE**

1. Composés de formule I

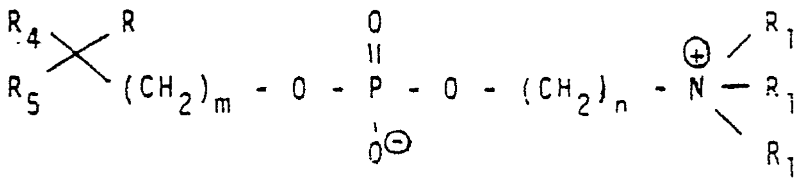

dans laquelle
R représente l'hydrogène ou un groupe alkyle en $C_{14}$–$C_{18}$ ou $CH_2OR_2$ ou $CH_2CH_2OR_2$, où $R_2$ représente un groupe alkyle en $C_{14}$–$C_{18}$
chaque $R_1$ représente indépendamment un groupe alkyle en $C_1$–$C_3$,
m signifie 1 ou 2,
n signifie un nombre entier de 2 à 8 et
$R_4$ et $R_5$ représentent ensemble avec l'atome de carbone adjacent

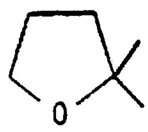

(a)

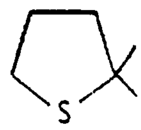

(b)

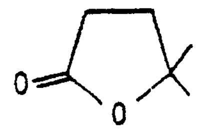

(c)

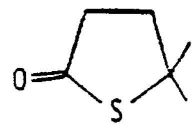

(d)

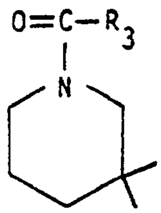

ou (e)

où $R_3$ représente un groupe alkyle ou alcoxy, contenant chacun de 12 à 24 atomes de carbone, et lorsque $R_4$ et $R_5$ représentent ensemble avec l'atome de carbone adjacent (i) un cycle e), R signifie H et (ii) un cycle autre que le cycle e), R a une signification autre que H.

2. Un composé selon la revendication 1, dans lequel chaque $R_1$ est identique et $R_4$ et $R_5$ représentent ensemble avec l'atome de carbone adjacent un cycle a); b); c); ou d).

3. Un composé selon la revendication 1, dans lequel chaque $R_1$ est identique et $R_4$ et $R_5$ représentent ensemble avec l'atome de carbone adjacent un cycle e) et m signifie 1.

4. Un composé selon la revendication 1, choisi parmi ceux dans lesquels $R_4$ et $R_5$ représentent ensemble avec l'atome de carbone adjacent un cycle a); b); c); ou d) et

i) R signifie R' et représente un groupe n-alkyle en $C_{16}$–$C_{18}$, $CH_2OR_2'$ ou $CH_2CH_2OR_2'$ où $R_2'$ signifie un groupe n-alkyle en $C_{16}$–$C_{18}$ et n signifie un nombre entier de 2 à 6,

ii) R signifie R″ et représente un groupe n-alkyle en $C_{16}$–$C_{18}$ ou $CH_2OR_2'$ où $R_2'$ est tel que défini sous (i) et n signifie un nombre entier de 2 à 4,

iii) R est tel que défini sous (ii), m signifie 1, n signifie 2 et chaque $R_1$ signifie $CH_3$ ou $R_4$ et $R_5$ représentent ensemble avec l'atome de carbone adjacent un cycle e) et

i) $R_3$ signifie $R_3'$ et représente un groupe alkyle ou alcoxy, ayant chacun de 12 à 20 atomes de carbone, m signifie 1 et n signifie un nombre entier de 2 à 6,

ii) $R_3$ signifie $R_3''$ et représente un groupe alkyle ou alcoxy, ayant chacun de 12 à 18 atomes de carbone, m signifie 1 et n signifie un nombre entier de 2 à 4,

iii) $R_3$ signifie $R_3'''$ et représente un groupe alcoxy ayant de 12 à 18 atomes de carbone, m signifie 1 et n signifie un nombre entier de 2 à 4 et les autres symboles sont tels que définis à la revendication 1.

5. Le 4-oxyde du sel interne de l'hydroxyde de 2-[hydroxy-(tétrahydro)-2-(octadécyl-5-oxo-2-furannyl)méthoxy-phosphinyloxy]-N,N,N-triméthyléthanaminium.

6. Le 4-oxyde du sel interne de l'hydroxyde de 2-[(2-octadécyloxyméthyltétrahydro-2-furannylméthoxy)-hydroxyphosphinyloxy]-N,N,N-triméthyl-éthanaminium.

7. Le 4-oxyde du sel interne de l'hydroxyde de 2-hydroxy-[1-octadécyloxycarbonyl-3-pipéridinyl-méthoxy]-phosphinyloxy]-N,N,N-triméthyléthanaminium.

8. Une composition pharmaceutique, comprenant un composé selon l'une quelconque des revendications 1 à 7 ensemble avec un véhicule ou diluant pharmaceutiquement acceptable.

9. Un composé selon l'une quelconque des revendications 1 à 7 pour l'utilisation comme médicament.

10. Un composé selon l'une quelconque des revendications 1 à 7 pour l'utilisation comme agent anti-tumoral.

11. Un procédé de préparation d'un composé selon la revendication 1, qui comprend

a) la réaction d'un composé de formule <u>C</u>

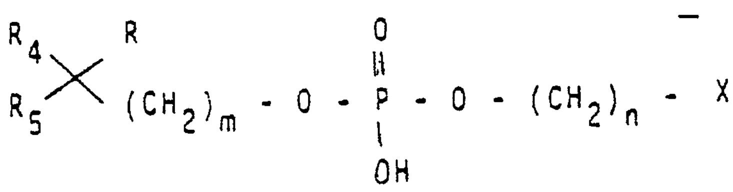

C

avec un composé de formule CC

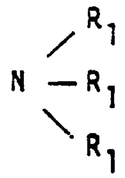

CC

dans lesquelles R, $R_1$, $R_4$, $R_5$, m et n sont tels que définis plus haut et X signifie un groupe éliminable, ou
b) lorsque n signifie 2
la réaction d'un composé de formule D

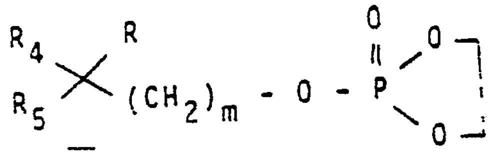

D

avec un composé de formule CC

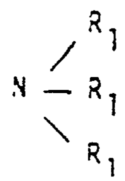

CC

dans lesquelles R, $R_1$, $R_4$, $R_5$ et m sont tels que définis plus haut.

**Revendications pour l'Etat Contractant AT**

1. Un procédé de préparation des composés de formule I

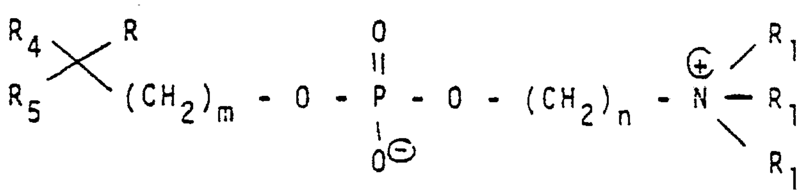

dans laquelle
R représente l'hydrogène ou un groupe alkyle en $C_{14}$–$C_{18}$ ou $CH_2OR_2$ ou $CH_2CH_2OR_2$, où $R_2$ représente un groupe alkyle en $C_{14}$–$C_{18}$,
chaque $R_1$ représente indépendamment un groupe alkyle en $C_1$–$C_3$
m signifie 1 ou 2,
n signifie un nombre entier de 2 à 8 et
$R_4$ et $R_5$ représentent ensemble avec l'atome de carbone adjacent

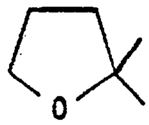 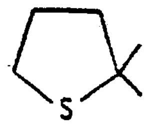 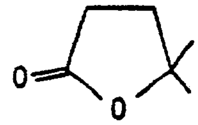 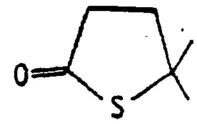

(a) (b) (c) (d)

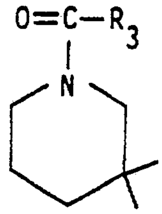

ou (e)

où $R_3$ représente un groupe alkyle ou alcoxy, contenant chacun de 12 à 24 atomes de carbone,
et lorsque $R_4$ et $R_5$ représentent ensemble avec l'atome de carbone adjacent (i) un cycle e), R signifie H et
(ii) un cycle autre que le cycle e), R a une signification autre que H,
procédé qui comprend

a) la réaction d'un composé de formule C

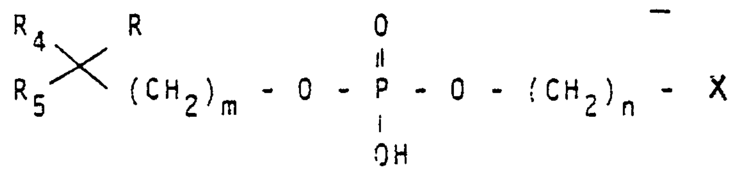

C

avec un composé de formule CC

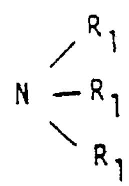

CC

dans lesquelles, R, $R_1$, $R_4$, $R_5$, m et n sont tels que définis plus haut et X signifie un groupe éliminable, ou

b) lorsque n signifie 2
la réaction d'un composé de formule D

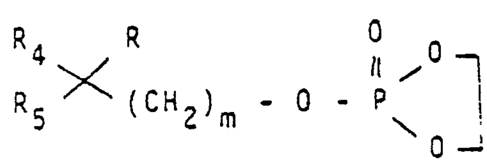

D

avec un composé de formule CC

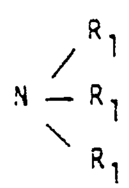

CC

dans lesquelles R, $R_1$, $R_4$, $R_5$ et m sont tels que définis plus haut.

2. Un procédé selon la revendication 1 pour la préparation du 4-oxyde du sel interne de l'hydroxyde de 2-[(2-octadécyloxyméthyltétrahydro-2-furannylméthoxy)-hydroxyphosphinyloxy]-N,N,N-triméthyl-éthanaminium, qui comprend

a) la réaction d'un composé de formule C, dans laquelle
R signifie un groupe octadécyloxyméthyle,
m signifie 1,
n signifie 2,
$R_4$ et $R_5$ représentent ensemble avec l'atome de carbone adjacent un groupe a) et
X signifie un groupe éliminable.
avec un composé de formule CC dans laquelle $R_1$ signifie un groupe méthyle ou
b) la réaction d'un composé de formule D dans laquelle R, m, $R_4$ et $R_5$ sont tels que défini dans cette revendication,
avec un composé de formule CC dans laquelle $R_1$ signifie un groupe méthyle.

3. Un procédé de préparation du composé selon la revendication 2, qui comprend la réaction du composé de formule D dans laquelle R, m, $R_4$ et $R_5$ sont tels que définis à la revendication 2, avec la triméthylamine.

4. Un procédé de préparation d'une composition pharmaceutique, qui comprend le mélange d'un composé de formule I tel que défini à la revendication 1 avec un véhicule ou diluant pharmaceutiquement acceptable.